# EUROPÄISCHE PATENTSCHRIFT

(11) **EP 1 145 009 B1**
(45) Veröffentlichungstag und Bekanntmachung des Hinweises auf die Patenterteilung: **06.05.2004**
(21) Anmeldenummer: 00905017.0
(22) Anmeldetag: 03.02.2000
(51) Int. Cl.: G01N 33/543

(54) **VERFAHREN ZUM NACHWEIS VON ANALYTEN IN EINER MESSPROBE SOWIE MESSTRÄGER HIERFÜR**
METHOD OF DETECTING ANALYTES IN A SAMPLE AND SUPPORT FOR THIS PURPOSE
PROCEDE DE DETECTION D'ANALYTES DANS UN ECHANTILLON POUR ESSAI ET SUPPORT DE MESURE Y RELATIF

(30) Priorität: 03.02.1999 DE 19904288; 17.08.1999 DE 19938839
(43) Veröffentlichungstag der Anmeldung: 17.10.2001
(73) Patentinhaber: EUROPÄISCHES LABORATORIUM FÜR MOLEKULARBIOLOGIE (EMBL), D-69117 Heidelberg (DE)
(72) Erfinder: BECKER, Peter, D-82194 Gröbenzell (DE); HÖRBER, Heinrich, D-91744 Weiltingen (DE)
(74) Vertreter: Prechtel, Jörg, Dipl.-Phys. Dr.
(86) Internationale Anmeldenummer: PCT/EP2000/000876
(87) Internationale Veröffentlichungsnummer: WO 2000/046600

(56) Entgegenhaltungen:
- WO-A-96/05326
- WO-A-96/09548
- WO-A-98/01533
- WO-A-98/12559
- T. SCHALKHAMMER: "Metal nano clusters as transducers for bioaffinity interactions" MONTASHEFTE FÜR CHEMIE/CHEMICAL MONTHLY, Bd. 129, Nr. 10, 1998, Seiten 1067-1092, XP000917114

## Beschreibung

Die Erfindung befaßt sich mit dem Nachweis von Analyten in einer Meßprobe. Als Analyten kommen beispielsweise Proteine, Peptide, Nukleinsäuren und deren Derivate sowie Moleküle, die an diesen binden können, in Frage.

Als Meßprobe kommt jede Probe in Frage, von der vermutet wird, daß sie mindestens einen Teil der gesuchten Analyten enthält. Es kann sich dabei z. B. um eine Blutprobe, eine Serumprobe oder/und eine Urinprobe handeln, oder allgemein um jede Lösung, die den gesuchten Analyten enthält.

Zum Nachweis von Analyten in einer Meßprobe ist der Einsatz sogenannter Bindungsassays bekannt. Bei solchen Bindungsassays werden im allgemeinen Analyten durch ihre spezifische Wechselwirkung mit Rezeptoren nachgewiesen. Beispiele für solche Wechselwirkungen sind Protein/Protein-Wechselwirkungen, die beispielsweise während der Genexpression auftreten, Enzym/Substrat- oder Enzym/Effektor-Wechselwirkungen, die im Stoffwechsel auftreten, oder Protein/DNA- oder Protein/RNA-Wechselwirkungen während der Genexpression. Ein systematisches Verständnis dieser molekularen Wechselwirkungen ist eine Grundvoraussetzung für das Verständnis aller biologischen Vorgänge sowohl in normalen als auch in erkrankten Zellen.

Weiterhin können mit Bindungsassays auch Nukleinsäuren nachgewiesen werden. DNA/DNA-Wechselwirkungen spielen eine Hauptrolle in der Molekularbiologie, insbesondere bei der Identifizierung von Genen und bei Strategien zur Kartierung von DNA, beim Nachweis und der Quantifizierung der Genexpression sowie bei der molekularen Diagnose oder Therapie von Erkrankungen. In Frage kommen selbstverständlich auch DNA/RNA- und RNA/RNA-Wechselwirkungen.

Aufgrund der großen Anzahl von Genen, Proteinen, chemischen Effektoren oder RNA-Aptameren erfordert ein Verstehen oder Eingreifen in biochemische Prozesse häufig die Erkennung, Quantifizierung oder Klassifizierung einer Vielzahl von molekularen Wechselwirkungen oder die Identifizierung von wirksamen Wechselwirkungspartnern aus einer großen Anzahl von möglichen Kombinationen. Das Screening und Analysieren einer großen Anzahl von molekularen Wechselwirkungen ist bei den im Stand der Technik bekannten Verfahren jedoch häufig begrenzt.

Meßproben sollen oftmals auf eine Vielzahl von Analyten hin untersucht werden. Dabei ergibt sich das Problem, daß bei der Auswertung große Datenmengen anfallen. Um in der Praxis erfolgreich zu bestehen, muß die Auswertung jedoch in vertretbarer Zeit möglich sein. Bekannte Analysesysteme haben sich hier als nur bedingt zufriedenstellend erwiesen.

Aus einem Artikel "Metal Nano Clusters as Transducers for Bioaffinity Interactions" von Thomas Schalkhammer in Monatshefte für Chemie 000,1-26, Springer-Verlag 1998, Seiten 1-26, ist ein Sensoraufbau für biorekognitive Bindungsvorgänge und katalytisch-enzymatische Prozesse bekannt, bei dem auf einem Polycarbonat-Substrat eine Spiegelschicht aus Silber und darüber eine Abstandsschicht aus einem Polymermaterial angeordnet sind. Die Abstandsschicht dient ihrerseits als Träger für die darauf immobilisierten Binder. Die Auswertung erfolgt mit Hilfe von Metall-Clustern, mit denen die nachzuweisenden Analyten markiert werden und die in elektromagnetische Wechselwirkung mit der metallischen Spiegelschicht treten. In Sensorbereichen starker Überdeckung mit gebundenen Metall-Clustern wird Licht, das von der dem Polycarbonat-Substrat abgewandten Seite der Spiegelschicht her eingestrahlt wird, stärker absorbiert als in überdeckungsfreien oder schwächer überdeckten Sensorbereichen, was die optische Auslesung des Sensors über Absorptionsmessungen ermöglicht.

Für die Funktion dieses bekannten Sensors ist die Dicke der Abstandsschicht ein entscheidender Parameter. Diese muß in einem vorgegebenen Bereich gewählt werden, der von der Wellenlänge des bei der späteren Auswertung eingestrahlten Lichts abhängt. Es muß insbesondere sichergestellt sein, daß die Abstandsschicht auf ihrer gesamten Ausdehnung eine gleichmäßige Dicke besitzt. Hierfür ist ein hoher herstellungstechnischer Aufwand zu betreiben.

In dem angesprochenen Artikel werden für die optische Auswertung dieses Sensors schon Lesegeräte vorgeschlagen, die es erlauben, auch größere Datenmengen zu bewältigen, unter anderem CD-ROM-Lesegeräte.

Aus WO96/09548 ist ein kreisscheibenförmiger Meßträger bekannt, auf dessen Oberfläche ein analytspezifischer Binder immobilisiert ist. Eingebaut in den Meßträger ist eine Reflexionsschicht; im übrigen besteht er aus einem optisch transparenten Material. Zum Nachweis von an den Bindern anhaftenden Analyten dient ein optisches Lesegerät, welches mit einem Laserstrahl die mit den Bindern versehene Oberfläche des Meßträgers spiralförmig abtastet.

Aus WO98/12559 ist ebenfalls ein kreisscheibenförmiger Meßträger bekannt, auf dessen Oberfläche analytspezifische Binder in einer Vielzahl von Nachweisfeldern immobilisiert sind, welche längs Kreis- oder Spiralspuren verteilt sind. Die Nachweisfelder sind auf einer Substratschicht des Meßträgers angeordnet, auf dessen gegenüberliegender Seite sich eine Reflexionsschicht befindet. Zum Auslesen der Nachweisfelder dient wiederum ein Laserstrahl, der wie bei einem CD-ROM-Laufwerk die die Nachweisfelder tragende Seite des Meßträgers abtastet.

Aus WO98/01533 ist es zudem bekannt, in Nachweisfeldern auf einem kreisscheibenförmigen Meßträger befindliche Bindemoleküle mit magnetischen Markern zu markieren und die Nachweisfelder mittels eines magnetischen Leseverfahrens auszuwerten.

Aufgabe der Erfindung ist es, einen Weg aufzuzeigen, wie mit geringem Aufwand, nicht nur was die datentechnische Auswertung anbelangt, sondern auch was die Bereitstellung des Sensors anbelangt, eine Meßprobe auf eine große Fülle von Analyten hin untersucht werden kann.

Zur Lösung dieser Aufgabe ist erfindungsgemäß ein Verfahren gemäß Anspruch 1 vorgesehen.

Bei der Erfindung können die Nachweisfelder direkt auf dem Substrat ausgebildet werden. Hierbei kann es sich empfehlen, die Substratoberfläche zunächst chemisch vorzubehandeln oder durch Vorbehandlung mit einem Sauerstoffplasma eine Oberflächenmodifizierung zu bewirken. Diese Modifikation kann das Aufbringen der Binder zur Ausbildung der analytspezifischen Nachweisfelder erleichtern. Unterhalb der Binder ist bei der Erfindung demnach keine Abstandsschicht mit einer präzise einzuhaltenden Dicke auf das Substrat aufzubringen, wie dies bei dem von Schalkhammer bekannten Sensor zwingend erforderlich ist. Insofern ergibt sich ein reduzierter fertigungstechnischer Aufwand.

Die Reflexionsschicht wird für die optische Auswertung des Assays benötigt. Sie wird nach Abschluß der molekularbiologischen Verfahrensschritte auf das Substrat über den Nachweisfeldern aufgebracht . Aufgrund ihres guten Reflexionsvermögens empfehlen sich metallische Werkstoffe. Grundsätzlich sind auch andere Materialien denkbar, etwa Dielektrika. Bevorzugtwird die Reflexionsschicht aus Aluminium gebildet. Denkbar ist auch Silber als Material für die Reflexionsschicht. Die Reflexionsschicht kann durch chemisches Aufdampfen ausgebildet werden. Möglich ist aber auch, eine vorgefertigte reflektierende Folie auf das Substrat aufzukleben.

Die optische Transparenz des Substrats macht es zusammen mit der auf der substratfernen Seite der Nachweisfelder angeordneten Reflexionsschicht möglich, zur optischen Auswertung dieses erfindungsgemäßen molekularbiologischen Sensors auf die Hardware handelsüblicher CD-Lesegeräte zurückzugreifen. Insbesondere bei spiraliger Anordnung der Nachweisfelder können solche handelsüblichen CD-Lesegeräte mit vergleichsweise geringem Aufwand so modifiziert werden, daß aus dem rückkehrenden Licht des die Nachweisfelder abtastenden Laserstrahls die gewünschten Informationen herausgefiltert werden können. Denkbar ist es, daß hierzu lediglich Software-Modifikationen erforderlich sind. Selbst bei kreisförmiger Anordnung der Nachweisfelder ist die Technik solcher CD-Lesegeräte grundsätzlich anwendbar, sofern die konstruktiven und softwaremäßigen Voraussetzungen für einen Sprung des die Nachweisfelder abtastenden Laserstrahls von Kreislinie zu Kreislinie geschaffen werden.

Besonders eignet sich ein CD-ROM-Lesegerät, das bei Einbettung in eine Computerarchitektur dem Anwender die Möglichkeit gibt, die Auswerte-Software selbst zu definieren.

Die Auswertung der Nachweisfelder kann abhängig vom gewählten Auswertealgorithmus quantitative wie auch qualitative Aussagen liefern. Mittels geeigneter Marker können die optischen Eigenschaften, insbesondere das Absorptionsverhalten, von Nachweisfeldern, in denen sich Analyteri an Rezeptoren angeheftet haben, von Nachweisfeldern, an deren Rezeptoren sich keine Analyten angeheftet haben, unterscheidbar gemacht werden. Auf _diese Weise ist es beispielsweise über Absorptionsmessungen möglich, diejenigen Nachweisfelder zu erkennen, die nach Abschluß der molekularbiologischen Verfahrensschritte Analyten tragen. Dies wäre zunächst eine rein qualitative Aussage. Will man darüber hinaus auch quantitative Aussagen treffen, so ist es denkbar, die Nachweisfelder mehrmals abzutasten und eine Schwelle, die bei der Auswertung als Entscheidungsgrenze zwischen Vorhandensein und Nichtvorhandensein von Analyten genommen wird, von Abtastdurchgang zu Abtastdurchgang schrittweise zu verändern. Auf diese Weise können auch Aussagen über die Menge der in den einzelnen Nachweisfeldern hängengebliebenen Analyten getroffen werden.

Wenn der Assay mit einem ggf. geeignet angepaßten CD-ROM-Laufwerk ausgelesen wird, ermöglicht ein angeschlossener Computer dem Anwender die Weiterverarbeitung der von dem Laufwerk gelieferten Datenmenge, wobei er das Ergebnis dieser Weiterverarbeitung auf externen Datenspeichern abspeichern kann, beispielsweise um Datenbanken über Patienten zu erstellen. Zur Datenspeicherung können herkömmliche Standardspeicher, etwa Festplatten oder Floppy-Discs, verwendet werden. Mit Computerhilfe ist eine schnelle Auswertung der Datenflut des Laufwerks möglich. Die Kapazität herkömmlicher Computer und Speichersysteme ist in der Regel so ausreichend, daß auch komplexe Genanalysen darauf durchgeführt werden können.

In der Regel wird es erwünscht sein, scharf definierte Nachweisfelder zu erhalten, die zusätzlich sehr klein sein sollen, um eine hinreichende Zahl von Nachweisfeldern auf der Substratscheibe unterzubringen, die zweckmäßigerweise die Größe einer herkömmlichen CD (Compact Disc) besitzt. Wenn man davon ausgeht, daß der radiale Abstand zweier aufeinanderfolgender Spiralwindungen zweckmäßigerweise etwa 1,6 µm beträgt, so empfiehlt es sich, die Binder in Feldern aufzubringen, die radial nicht breiter als 1 µm sind, damit zwischen bezogen auf die Scheibenachse radial benachbarten Nachweisfeldern ein hinreichender Abstand besteht und jedes einzelne Nachweisfeld als solches ortsaufgelöst werden kann. Bevorzugt sind die Nachweisfelder radial sogar schmäler als 1 µm, wobei es zweckmäßig sein kann, sie mit einer radialen Breite auszubilden, die im wesentlichen der Breite der bei herkömmlichen CDs eingeprägten Datenvertiefungen (sogenannte Pits) entspricht, nämlich etwa 0,5 µm. Gleichfalls empfiehlt es sich, entlang der Spirallinie bzw. einer Kreislinie einander benachbarte Nachweisfelder mit Abstand voneinander anzuordnen, um deren individuelle Auflösung durch das Auswertesystem zu ermöglichen. Die Nachweisfelder können als im wesentlichen quadratische oder kreisförmige Flächen ausgebildet werden. Denkbar ist auch, sie entsprechend der Gestalt der Datenpits herkömmlicher CDs in Umfangsrichtung, also in Spiral- bzw. Kreislinienrichtung, länglich auszubilden, etwa oval oder rechteckig.

Um die Binder unter den vorstehend skizzierten dimensionellen Randbedingungen für die Nachweisfelder auf das Substrat aufzubringen, eignen sich insbesondere Mikroprinttechniken, Tintenstrahltechniken oder Elektrospraytechniken. Zum Nachlesen, was Mikroprinttechniken anbelangt, wird beispielhaft verwiesen auf: "Microfabrication, Microstructures and Microsystems" von Dong Qin et al., "Topics In Current Chemistry", Band 194, 1998, Seite 6ff., Springer-Verlag. Bezüglich Tintenstrahltechniken wird beispielhaft verwiesen auf: "A new device for multifunctional dosage of liquids by a free jet" von N. Hey et al., Proceedings IEEE-Mems 1998, CH 36176. Elektrospraytechniken, insbesondere Nanoelektrospraytechniken, können beispielsweise in "Analytical Properties of the Nanoelectrospray Ion Source" von M. Wilm und M. Mann in Analytical Chemistry, Band 68, Nr. 1, 1. Januar 1996, Seiten 1-8, sowie in "Electrospray and Taylor-Cone theory, Dole's beam of macromolecules at last?" von M. Wilm, M. Mann in International Journal of Mass Spectrometry and Ion Processes 136 (1994), Seiten 167-180, nachgelesen werden. Diese Techniken ermöglichen es, die Nachweisfelder mit hoher Ortsauflösung gezielt mit vorbestimmten Bindern zu versehen. Die Binder können auf die Außenoberfläche der Substratscheibe aufgebracht werden. Denkbar ist es auch, in Analogie zu den Datenpits herkömmlicher CDs kleine Vertiefungen in die Außenoberfläche des Substrats einzubringen und darin die analytspezifischen Binder zu immobilisieren. Falls solche Vertiefungen für die Binder vorgesehen werden, wird es zweckmäßig sein, diese mit einer radialen Breite von etwa 0,5 µm auszubilden, entsprechend der Breite der Datenpits herkömmlicher CDs.

Um biologische Wechselwirkungen auf molekularer Ebene zu untersuchen, kommen als Analyten insbesondere Nukleinsäuren oder/und Proteine in Betracht. So kann das erfindungsgemäße Verfahren verwendet werden, um Protein/Protein-Wechselwirkungen, wie sie beispielsweise während der Genexpression oder als Zellsignale auftreten, Enzym/Substrat- oder Enzym/Effekt-Wechselwirkungen während des Stoffwechsels oder Protein/DNA- oder Protein/RNA-Wechselwirkungen während der Genexpression nachzuweisen.

Durch geeignete Wahl der Binder in den Nachweisfeldern können auch DNA/DNA-Wechselwirkungen nachgewiesen werden, wie es insbesondere zur Genidentifizierung und zur Kartierung von Genen, beim Untersuchen und Quantifizieren der Genexpression sowie bei der molekularen Diagnose und/oder Therapie von Erkrankungen von Bedeutung ist.

Molekulare Wechselwirkungen und Enzymaktivitäten können durch die Bindung von RNA-Aptameren oder kleinen chemischen Verbindungen an Makromoleküle beeinflußt werden. Natürliche und synthetische Effektormoleküle erlauben es deshalb häufig, biochemische Verfahren durch Modulation von oder Eingreifen in makromolekulare Wechselwirkungen zu manipulieren.

Bevorzugt handelt es sich bei dem Substratmaterial um ein nicht poröses Material. Die Verwendung eines nicht porösen Trägers ermöglicht das definierte Aufbringen auch kleiner Nachweisfelder, so daß eine Miniaturisierung des Testformats bzw. das Aufbringen einer Vielzahl von Nachweisfeldern möglich ist. Geeignete Materialien für das scheibenförmige Substrat sind z. B. Kunststoffe und Glas. Für CD-ROM-Laufwerksapplikationen handelt es sich bevorzugt um ein fokussierendes Material, insbesondere um Polycarbonat.

Die Immobilisierung der Binder an den Nachweisfeldern kann nach an sich bekannten Verfahren durchgeführt werden. Die Immobilisierungsstrategie hängt von der Art des zu immobilisierenden Moleküls und des jeweiligen Substrats ab. Im allgemeinen können Binder direkt an eine Matrix über eine chemische Reaktion gebunden werden, beispielsweise über eine spezielle Aminosäure in einem Protein (insbesondere Cystein oder Lysin) oder über das Phosphatgrundgerüst einer DNA. Die Immobilisierung kann auch mit bifunktionellen chemischen Quervernetzern oder über eine spezifische hochaffine Wechselwirkung, wie etwa die Biotin/Streptavidin-Wechselwirkung durchgeführt werden. Die analytspezifischen Binder können auf der Oberfläche auch adsorbiert werden, wobei eine kovalente Bindung jedoch bevorzugt ist. Als Binder werden auf die Oberfläche des Substrats Substanzen oder/und Moleküle aufgebracht, die in der Lage sind, den gewünschten Analyten spezifisch, insbesondere mit hoher Affinität, zu binden.

Nach Inkontaktbringen der Meßprobe mit den Nachweisfeldern wird das Vorhandensein oder/und die Menge der nachzuweisenden Analyten durch optische Auswertung der Nachweisfelder ermittelt. Hier können alle dem Fachmann bekannten Methoden herangezogen werden. Ein übliches Verfahren, um molekulare Wechselwirkungen zu analysieren, besteht aus mehreren Schritten: Ein erster Wechselwirkungspartner, z. B. ein analytspezifischer Binder oder Rezeptor, wird mit einem festen Träger kovalent oder adsorptiv verknüpft. Beim Inkontaktbringen der Meßprobe mit den Nachweisfeldern kann ein zweiter Wechselwirkungspartner, z. B. der Analyt, mit dem Rezeptor wechselwirken. Anschließend wird das Vorhandensein des Analyten (oder das Nichtvorhandensein in kompetitiven Testformaten) an der Stelle, an der der Rezeptor immobilisiert ist, nachgewiesen. Die Wechselwirkung zwischen den zwei Bindepartnern wird bevorzugt unter Bedingungen durchgeführt, bei denen beide Reaktanten in einer nativen, aktiven Konfiguration vorliegen, bevorzugt in einer flüssigen Reaktion. Besonders bevorzugt wird das Inkontaktbringen unter einem annähernd physiologischen pH-Wert und bei ionischen Bedingungen durchgeführt.

Der Nachweis erfolgt beim erfindungsgemäßen Verfahren durch Detektion einer Änderung der optischen Eigenschaften der Nachweisfelder. Die optische Änderung der Nachweisfelder kann z. B. durch Isotope, Enzyme, Fluorochrome, Farbstoffe, Metallkolloide, Beads oder ähnliches herbeigeführt werden, die als Markersystem dienen. Zum Nachweis wird einer der Wechselwirkungspartner, d. h. der Rezeptor oder der Analyt, direkt oder indirekt markiert. Bevorzugt werden zur Detektion Proteine oder Nukleinsäuren mit einer Biotineinheit derivatisiert, wonach eine Erkennung durch Streptavidinkonjugate möglich ist.

Bevorzugt resultiert das Nachweisverfahren in der Präzipitation eines Farbstoffs oder der Lokalisierung eines Fluorochroms an der Stelle der molekularen Wechselwirkung oder in der Anlagerung von Metall-Clustern mit starker elektromagnetischer Wechselwirkung. Latex-Beads oder Kunststoff-Beads können ebenfalls angebunden werden. Sie bewirken aufgrund ihres Brechungsverhaltens und ihrer gekrümmten (sphärischen) Oberfläche eine Streuung des einfallenden Ausleselichts, die als Intensitätsminderung detektierbar ist. Bei geeigneter Dimensionierung der Beads können Effekte der destruktiven Interferenz erzielt werden.

Die wechsetwirkenden Moleküle, also der Rezeptor und der Analyt, können entweder direkt über ihre spezifischen Bindestellen nachgewiesen werden oder indirekt, indem sie mit einem Marker versehen werden, der eine bekannte spezifische Bindestelle enthält. Beispiele für solche Marker sind Epitope, für die monoklonale Antikörper bekannt sind, oder eine Biotingruppe, die mit Streptavidin bindefähig ist. Daneben ist auch das direkte spezifische Binden eines Antikörpers an den Analyten möglich. Antikörper und Streptavidin werden bevorzugt mit einem Enzym konjugiert, um eine Auswertung der Nachweisfelder zu ermöglichen. Beispiele für bevorzugte Enzyme sind Meerrettichperoxidase, alkalische Phosphatase und β-Galaktosidase. Bei Zugabe geeigneter Substrate läuft eine enzymatische chromogene Reaktion ab, wobei gefärbte Produkte entstehen, die an den Orten präzipitieren, an denen die Enzyme gebunden sind und somit das Vorhandensein des Analyten anzeigen. Geeignete Substrate der obigen Enzyme, die eine optische Auswertung ermöglichen, sind für Meerrettichperoxidase beispielsweise Diaminobenzidin (DAB), welches ein in Wasser und Ethanol unlösliches braunes Produkt ergibt, DAB+ Metall, welches in Gegenwart von Kobalt oder Nickel ein graues bis schwarzes unlösliches Produkt ergibt, Chlornaphtol, das eine blau-schwarze, wasserlösliche Färbung ergibt, Aminoethylcarbazol, das ein rotes, wasserunlösliches Produkt ergibt. Bevorzugte Substrate für alkalische Phosphatase sind Naphthol-AS-BI-Phosphat/New-Fuchsin, was ein rotes, unlösliches Produkt ergibt, Bromchlorindolylphosphat/Nitrotetrazolium, was ein schwarzviolettes Präzipitat ergibt, und für β-Galaktosidase Bromchlorindolyl-b-D-Galaktpyranosit (BCIG), was ein unlösliches blaues Produkt ergibt. Der Nachweis der Analyten kann hier leicht durch optische Detektion des gefärbten Bereichs erfolgen.

Eine weitere Möglichkeit zum Nachweis der Wechselwirkungsstellen ist die Anfärbetechnik mit Goldkolloiden, wobei auch andere Typen von Metallclustern bzw. -Beads verwendet werden können. Hier sind insbesondere Silberteilchen vorteilhaft, die die Sensitivität eines optischen Nachweissystems aufgrund nichtlinearer optischer Effekte nahe einer Metalloberfläche der Reflexionsschicht erhöhen können. Weiterhin bevorzugt ist die Verwendung von Farbstoffen als Marker, insbesondere von gefärbten Latexpartikeln. Es können auch Glas-Beads aus Siliziumoxid verwendet werden, die mit einem Farbstoff in hoher Konzentration gefüllt sind.

Neben der Messung der Absorption ist bei Wahl geeigneter Marker, beispielsweise fluoreszierender Stoffe, auch eine Messung der Fluoreszenz möglich, wobei hier die Detektionswellenlänge eine andere als die eingestrahlte Wellenlänge ist. Sofern marktgängige CD-Laufwerke, insbesondere CD-ROM-Laufwerke, bei der Auswertung herangezogen werden sollen, können im Fall von Fluoreszenzmessungen konstruktive Laufwerksmodifikationen neben einer entsprechenden Anpassung der Software erforderlich sein. Insbesondere kann es notwendig sein, einen speziell auf die Fluoreszenzwellenlänge abgestimmten Detektor einzubauen.

Die Erfindung ermöglicht es, auf der die Nachweisfelder tragenden Flachseite des Substrats zusätzliche Informationen einzuschreiben, die zeitgleich mit den Nachweisfeldern ausgelesen und ausgewertet werden können. Demgemäß wird vorgeschlagen, daß auf der die Nachweisfelder tragenden Flachseite des Substrats entlang der Spirallinie bzw. mindestens einer Kreislinie zusätzlich Datenfelder ausgebildet werden, welche meßprobenbezogene oder/und nachweisfeldbezogene oder/und auswertungsbezogene Informationen enthalten. Bei den meßprobenbezogenen Informationen kann es sich beispielsweise um Informationen über Ort und Zeit der Gewinnung der Meßprobe, über die Art der Meßprobe oder über die Person handeln, der die Meßprobe entnommen wurde. Die nachweisfeldbezogenen Informationen können molekularbiologische oder biochemische Angaben über die Nachweisfelder enthalten, insbesondere über den Bindertyp der einzelnen Nachweisfelder. Die auswertungsbezogenen Informationen können Angaben über das zum Nachweis der Analyten angewendete Detektionsprinzip, etwa enzymatische Detektion, Detektion über Farbstoffe, Detektion über Metall-Cluster oder dergleichen, enthalten. Ferner können sie Angaben darüber enthalten, welches physikalische Abtastprinzip ein Lesegerät anwenden soll, etwa eine Absorptionsmessung oder eine Fluoreszenzmessung. Zudem können die auswertungsbezogenen Informationen dem Lesegerät Lage und Ort der Nachweisfelder auf dem Substrat angeben und ihm damit signalisieren, wann das Lesegerät von einer Software zum Lesen der Datenfelder auf eine Software zum Lesen der Nachweisfelder umschalten soll. Insbesondere ist denkbar, daß die auswertungsbezogenen Informationen schon zumindest Teile einer Auswerte-Software enthalten, die vom Lesegerät bei der Auswertung der Nachweisfelder abgearbeitet wird. Auf diese Weise kann der Anwender von mühsamen Programmierungsaufgaben zur Softwareanpassung seines Computerarbeitsplatzes entlastet werden. Die vollständige Auswerte-Software kann bereits herstellerseitig, soweit geeignete Software-Standards existieren, in den Sensor eingeschrieben werden.

Grundsätzlich ist es denkbar, die Nachweisfelder und die Datenfelder längs der Spirallinie getrennt anzuordnen. Es kann aber zweckmäßig sein, Nachweisfelder und Datenfelder abwechselnd entlang der Spirallinie anzuordnen, beispielsweise in der Weise, daß einem einzelnen Nachweisfeld oder einer Gruppe von Nachweisfeldern ein Datenfeld zugeordnet wird, das dem Nachweisfeld bzw. der Gruppe von Nachweisfeldern längs der Spirallinie vorhergeht und dem Auswertesystem Informationen über dieses Nachweisfeld bzw. diese Gruppe von Nachweisfeldern liefert.

Sofern die Nachweisfelder längs konzentrischer Kreislinien angeordnet werden, können Nachweisfelder und Datenfelder ebenfalls abwechselnd entlang mindestens einer Kreislinie angeordnet werden. Denkbar ist aber auch, daß Nachweisfelder und Datenfelder jeweils auf gesonderten Kreislinien ausgebildet werden.

Die Datenfelder sollen zweckmäßigerweise von der gleichen Seite der Substratscheibe her ausgelesen werden können wie die Nachweisfelder. Dabei besteht eine Möglichkeit darin, zur Bildung der Datenfelder Vertiefungen in die Nachweisfelder tragende Flachseite des Substrats einzubringen, wobei die Reflexionsschicht so angebracht wird, daß sie in die Vertiefung hineinreicht. Zweckmäßigerweise erfolgen die Codierung der Daten und die Anordnung der Vertiefungen nach Maßgabe eines gängigen CD-Formats, insbesondere des CD-ROM-Formats, so daß die Datenfelder mittels herkömmlicher CD-Laufwerke ohne Softwaremodifikationen gelesen werden können.

Es ist sogar denkbar, zur Bildung der Datenfelder auf Vertiefungen zu verzichten. Durch Anbindung von optisch absorbierenden oder streuenden Substanzen an das Substrat kann ebenfalls Einfluß auf die Reflexion des zur Auslesung auf die Nachweis- und Datenfelder gerichteten Lichtstrahls genommen werden. So ist es denkbar, bei geeigneter Wahl von Metall- oder Kunststoff-Beads ähnliche Effekte destruktiver Interferenz zu erzielen, wie sie auch durch Vertiefungen in der Substratoberfläche erreicht werden. Es kann daher vorgesehen sein, daß zur Bildung der Datenfelder eine einfallendes Leselicht beeinflussende Substanz auf die die Nachweisfelder tragende Flachseite des Substrats aufgebracht wird.

Weil die Reflexionsschicht erst nach Abschluß sämtlicher molekularbiologischer oder biochemischer Analyseschritte aufgebracht wird, sind Vorher-Nachher-Messungen der Nachweisfelder nicht möglich, also Messungen vor und nach Inkontaktbringung der Meßprobe mit den Nachweisfeldern. Um dennoch aus den Meßsignalen verläßliche Aussagen darüber zu gewinnen, ob und welche Nachweisfelder mit Analyten besetzt sind, kann es vorteilhaft sein, auf der die Nachweisfelder tragenden Flachseite des Substrats entlang der Spirallinie bzw. mindestens einer Kreislinie zusätzlich mindestens ein Referenzfeld auszubilden, dessen optische Eigenschaften als Referenz bei der Auswertung der Nachweisfelder verwendet werden. Beispielsweise können diese Referenzfelder einen bekannten, für analytfreie Nachweisfelder typischen Referenzabsorptionsgrad für das Licht des abtastenden Lichtstrahls besitzen, der von einem Absorptionsgrad unterscheidbar ist, den analytbehaftete Nachweisfelder typischerweise besitzen. Daneben können auch Referenzfelder ausgebildet sein, die den Analyten oder/und Marker enthalten und die bei korrekter Durchführung des Verfahrens als positive Kontrolle dienen. Die Referenzfelder können zudem zur Kalibrierung und zur Quantifizierung der Messungen herangezogen werden.

Im Bereich der Nachweisfelder kann sich eine verminderte Haftung der Reflexionsschicht einstellen, wenn diese unmittelbar auf die darunterliegende molekularbiologische oder biochemische Ebene aufgebracht wird. In diesem Fall kann es günstig sein, nach Inkontaktbringung der Meßprobe mit den Nachweisfeldern zunächst eine Deckschicht aus einem optisch transparenten Material auf die Nachweisfelder aufzubringen, bevor die Reflexionsschicht aufgebracht wird. Die Deckschicht kann auch zur Fixierung der Reagenzien in den Nachweisfeldern dienen. Das Material und die Dicke der Deckschicht wird man so aufeinander abstimmen, daß eine Beeinflussung der optischen Eigenschaften des Sensors durch die Deckschicht, etwa durch Fokussierungs- oder Absorptionseffekte, nicht oder zumindest in beherrschbarer Weise erfolgt. Ein Material auf Polymerbasis hat sich für die Deckschicht als geeignet gezeigt. Sie kann z. B. als Folie oder mittels eines Sprühverfahrens aufgebracht werden. Sofern der Sensor auch Vertiefungen in Datenfeldern aufweist, wird man sicherstellen müssen, daß die Vertiefungen nicht durch das Material der Deckschicht wieder aufgefüllt werden, um einen Verlust der durch die Vertiefungen repräsentierten Dateninformationen zu vermeiden. Sprühverfahren sind zur lokalen Aufbringung der Deckschicht denkbar. Vorstellbar ist es auch, die Oberfläche der Substratscheibe gedanklich in Segmente, etwa Kreissektoren, aufzuteilen, von denen ein Teil ausschließlich für Nachweisfelder und ein anderer Teil ausschließlich für Datenfelder reserviert wird. Die Segmente mit Datenfeldern können dann sehr leicht von der Deckschicht freigehalten werden.

Das Substrat mit den darauf immobilisierten Bindern kann als handelbare Einheit verpackt und an Anwender verschickt werden. Denkbar ist es, daß der Hersteller einen kundenspezifischen Satz von Bindern zusammenstellt und auf das Substrat aufbringt. Vorstellbar ist auch, kundenunspezifisch, aber applikationsspezifisch, beispielsweise für bestimmte Genanalysen, einen Satz von Bindern auf ein Substrat aufzubringen und dieses als vorbereiteten Meßträger anzubieten. Es empfiehlt sich, diesen Meßträger zu trocknen, bevor er verpackt und verschickt wird. Die zu untersuchende Meßprobe wird dann vom Anwender, also vom Käufer des Meßträgers, aufgebracht. Gleiches kann auch für markerenthaltende Reagenzien gelten. Die Aufbringung der Reflexionsschicht und ggf. der Deckschicht nach Abschluß des molekularbiologischen bzw. biochemischen Nachweisverfahrens, das auch Waschschritte umfassen kann, kann beim Anwender erfolgen, sofern diesem hierfür geeignete Geräte zur Verfügung stehen. Denkbar ist auch, daß der Anwender den analytbehafteten Meßträger zum Hersteller oder zu einem anderen Labor zurücksendet, wo diese Schichten aufgebracht werden.

Auf der Reflexionsschicht kann noch eine Schutzschicht flächig aufgebracht werden, wobei sich wegen seiner Schlag- und Kratzfestigkeit ein Material auf Acrylbasis eignet. Der so geschützte Meßträger kann über lange Zeiträume hinweg aufbewahrt werden und jederzeit wieder ausgelesen werden.

Die Erfindung betrifft ferner einen Meßträger gemäß Anspruchs 18, der zur Verwendung bei dem vorstehend erläuterten Verfahrens bestimmt ist.

Nach einem weiteren Aspekt ist erfindungsgemäß ein Verfahren gemäß Anspruch 23 vorgesehen.

Die kreis- oder spiralförmige Verteilung der Nachweisfelder auf dem Substrat ermöglicht die Verwendung marktgängiger Magnetplatten-Lesegeräte, sogenannter Hard-Disc-Laufwerke. Gegebenenfalls wird eine softwaremäßige Adaption der Laufwerkssteuerung notwendig sein. Die magnetische Auslesung der Nachweisfelder ermöglich es sogar, auf beiden Flachseiten des Substrats Nachweisfelder auszubilden, so daß sich die Packungsdichte der Sensoren mit Nachweisfeldern weiter erhöhen läßt. Hinsichtlich der gegenseitigen Abstände, der Größe und der Anordnung der Nachweisfelder sowie etwaiger Daten- und Referenzfelder auf dem Substrat treffen im wesentlichen die Angaben zu, die zuvor für den optisch auslesbaren Sensor gemacht wurden.

Um zu verhindern, daß die Marker durch Kontakt mit einem Lesekopf des Auswertegeräts vom Substrat weggerissen werden, kann nach Inkontaktbringung der Meßprobe mit den Nachweisfeldern eine Fixierschicht auf die Nachweisfelder aufgebracht werden. Diese wird man zweckmäßigerweise flächig auf jede Nachweisfelder tragende Flachseite des Substrats aufbringen. Für die Fixierschicht hat sich ein Material auf Polymerbasis als geeignet erwiesen.

Vor der Ausbildung der Nachweisfelder oder nach Inkontaktbringung der Meßprobe mitden Nachweisfeldern wird eine magnetische oder/und magnetisierbare Partikel enthaltende Magnetschicht flächig über jeder Nachweisfelder tragenden Flachseite des Substrats aufgebracht. Die Magnetschicht ermöglicht es, zusätzlich Daten in den Sensor einzuschreiben, die zusammen mit den Nachweisfeldern ausgelesen werden können. Die magnetischen Eigenschaften der Magnetschicht wird man so wählen, daß die durch die Marker hervorgerufenen lokalen Schwankungen der magnetischen Feldstärke oder der magnetischen Flußdichte nicht in folge der Magnetschicht "verschwimmen" und undetektierbar werden. Zwar ist es grundsätzlich denkbar, daß die Magnetschicht unterhalb der molekularbiologischen oder biochemischen Schicht des Sensors unmittelbar auf das Substrat aufgebracht wird. Zweckmäßig ist es jedoch, sie erst nach Abschluß der molekularbiologischen oderbiochemischen Verfahrensschritte aufzubringen, da sie so zugleich als Fixierschicht dienen kann.

Ein Testkit zur Durchführung des Verfahrens nach dem vorstehend beschriebenen ersten oder zweiten Aspekt umfaßt einen mit immobilisierten Bindern vorbereiteten Meßträger, Reagenzien zur Durchführung des Nachweisverfahrens, insbesondere optisch oder/und magnetisch detektierbare Nachweisreagenzien, sowie ggf. Wasch- oder/und Pufferlösungen. Der Meßträger liegt bevorzugt in getrockneter Form vor.

Schließlich betrifft die Erfindung die Verwendung eines Meßträgers der vorstehend beschriebenen Art in einem Immunoassay oder/und Nukleinsäurehybridisierungsassay oder/und Lektin-Zucker-Assay oder/und Protein-Nukleinsäure-Assay.

Die Erfindung wird im folgenden anhand der beigefügten Zeichnungen näher erläutert. Es stellen dar:
- Fig. 1: eine schematische Schnittdarstellung eines erfindungsgemäßen molekularbiologischen oder biochemischen Sensors ("Biosensor") mit Nachweis- und Datenfeldern und
- Fig. 2: schematisch die Verteilung der Nachweis- und Datenfelder auf dem Biosensor.

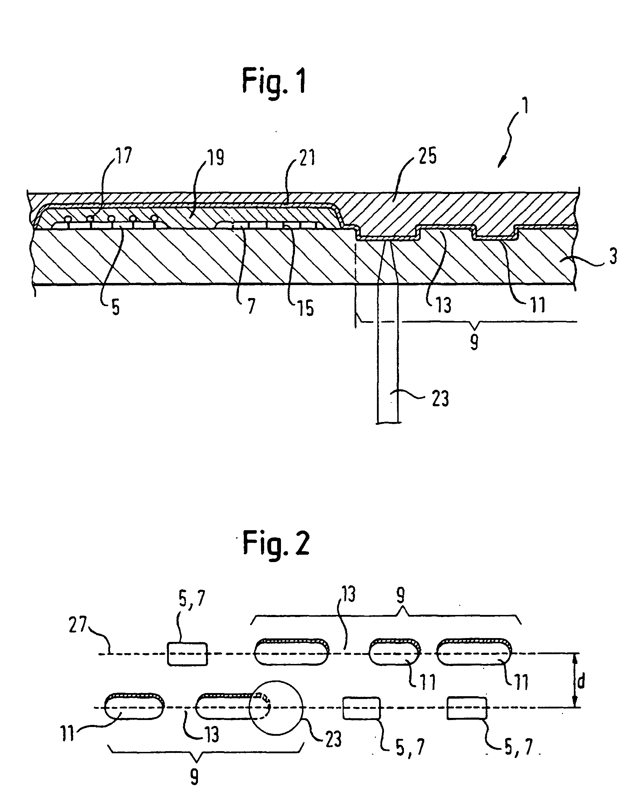

In Fig. 1, die die realen Größenverhältnisse nicht getreu wiedergibt, ist der Biosensor allgemein mit 1 bezeichnet. Er besitzt die Form einer Kreisscheibe. Seine Größe entspricht zweckmäßigerweise der einer herkömmlichen Compact Disc, deren Durchmesser üblicherweise etwa 12 cm beträgt. Der Biosensor 1 umfaßt ein Trägersubstrat 3, das aus einem optisch transparenten Material, vorzugsweise Polycarbonat, gefertigt ist. Auf der in Fig. 1 oberen Flachseite des Substrats 3 sind Nachweisfelder 5, 7 sowie Datenfelder 9 ausgebildet. In den Datenfeldern 9 sind Informationen über die Meßprobe oder/und über die Nachweisfelder oder/und über die Auswertung niedergelegt. Wie bei herkömmlichen CDs sind die Informationen in den Datenfeldern 9 durch abwechselnde vertiefte Bereiche 11 und nicht vertiefte Bereiche 13 des Substrats 3 repräsentiert.

In den Nachweisfeldern 5, 7 sind analytspezifische Binder bzw. Rezeptoren immobilisiert. Diese Binder sind in Fig. 1 schematisch durch kurze Striche 15 symbolisiert. Jedes Nachweisfeld 5, 7 trägt eine Vielzahl von Bindern 15 gleichen oder unterschiedlichen Typs. Das Nachweisfeld 7 repräsentiert in Fig. 1 ein Nachweisfeld, an dem nach Inkontaktbringung der Meßprobe mit den Nachweisfeldern 5, 7 keine Analyten haftengeblieben sind. Dagegen repräsentiert das Nachweisfeld 5 ein Nachweisfeld, an dem Analyten haftengeblieben sind. Diese Analyten sind in Fig. 1 schematisch durch kleine Kreise 17 dargestellt, die zugleich die Marker symbolisieren, anhand deren die optische Detektion des Vorhandenseins der Analyten möglich ist.

Die Nachweisfelder 5, 7 sind in eine Deckschicht 19 eingebettet, die nur in den die Nachweisfelder 5, 7 tragenden Bereichen des Substrats 3 aufgebracht ist, nicht jedoch in den die Datenfelder 9 tragenden Bereichen des Substrats 3. Die Deckschicht 19 besteht ebenfalls aus einem optisch transparenten Material, vorzugsweise einem Polymermaterial. Sie fixiert die Marker auf dem Substrat 3 und dient zugleich als Haftvermittler zwischen dem Substrat 3 und einer Reflexionsschicht 21, die flächig auf das Substrat 3 aufgebracht ist, über sämtliche Nachweisfelder 5, 7 und sämtliche Datenfelder 9 hinwegreicht und in die Vertiefungen 11 der Datenfelder 9 hineinreicht. Die Reflexionsschicht 21 besteht vorzugsweise aus Aluminium, kann aber beispielsweise auch aus Silber bestehen und wird zweckmäßigerweise durch chemisches Aufdampfen erzeugt. Die Reflexionsschicht 21 dient als Reflektor für das Licht eines Laserstrahls 23, der von der Unterseite des Substrats 3 her zur Auslesung der Nachweisfelder 5, 7 und der Datenfelder 9 auf den Biosensor 1 gerichtet wird. Das reflektierte Licht wird mittels gängiger Methoden ausgewertet. Beispielsweise wird es mittels eines Polarisationsfilters von dem eingestrahlten Licht getrennt und sodann auf seine Intensität hin ausgewertet. Das Material des Substrats 3 hat vorteilhafterweise einen solchen Brechungsindex, daßder Laserstrahl 23 auf seinem Hinweg im Substrat 3 fokussiert wird, so daß der letztlich auf die Nachweisfelder 5, 7 und die Datenfelder 9 einfallende Lichtfleck sehr klein gehalten werden kann.

Zur Oberseite hin ist der Biosensor 1 durch eine flächig aufgebrachte Deckschicht 25 abgeschlossen, die den Biosensor 1 vor schädlichen chemischen oder mechanischen Einflüssen schützt. Vorzugsweise besteht sie aus einem Acrylmaterial.

Die Nachweisfelder 5, 7 und die Datenfelder 9 sind in abwechselnder Reihenfolge längs einer Spirallinie auf dem Substrat 3 angeordnet. Fig. 2 zeigt einen Ausschnitt zweier aufeinanderfolgender Windungen der Spirallinie. Letztere ist in Fig. 2 als gestrichelte Linie dargestellt und mit 27 bezeichnet. Mit d ist ferner der radiale Abstand zwischen den beiden Windungen, also die Spiralsteigung, bezeichnet. Er beträgt beispielsweise etwa 1,6 µm. Die Vertiefungen 11, die bei Anwendung der für CDs, insbesondere CD-ROMs, gängigen Codierung abgestuft variable Umfangslänge besitzen können, sind beispielsweise etwa 0,5 µm in radialer Richtung breit. Um radiale Überlappungen mit den Nachweis- oder Datenfeldern benachbarter Spiralwindungen zu vermeiden, sind die Nachweisfelder 5, 7 ebenfalls radial so schmal ausgebildet, daß ein hinreichender Abstand zu den Feldern der benachbarten Spiralwindungen besteht. Insbesondere können die Nachweisfelder 5, 7 gleichfalls etwa 0,5 µm breit sein. Bei derartiger Bemessung kann ohne weiteres auch das "Ausfransen" in Kauf genommen werden, das bei enzymatischem Nachweis der in den Nachweisfeldern 5 anhaftenden Analyten häufig zu beobachten ist. In Umfangsrichtung können die Nachweisfelder 5, 7 länglich sein, so daß sie mit einer hinreichend großen Gesamtfläche ausgebildet werden können. Auch in Umfangrichtung werden die Nachweisfelder 5, 7 hinreichenden Abstand voneinander und von den Datenfeldern 9 aufweisen.

Die vorstehenden Maßangaben empfehlen sich insbesondere, wenn zur Auslesung der Daten- und Nachweisfelder CD-Laufwerke mit einem Fleckdurchmesser des Laserstrahls 23 von etwa 2 µm verwendet werden.

Nachfolgend werden noch einige molekularbiologische oder biochemische Anwendungsbeispiele der Erfindung erläutert.

### Beispiel 1

### Anwendungen

Die Anwendungen können gemäß der Natur des immobilisierten Bindepartners (A), des analytspezifischen Binders, und der Natur seines "Liganden", d. h. des Analyten (B), klassifiziert werden. Als Wechselwirkungspartner werden bevorzugt Nukleinsäuren und/oder Proteine verwendet, es können jedoch auch andere Partner von spezifischen Bindepaaren, wie etwa Lektine oder Zucker eingesetzt werden.

Bevorzugt werden die Rezeptoren bzw. Analyte, insbesondere Proteine und Nukleinsäuren, mit einer Biotingruppe derivatisiert, wobei die Biotingruppe dann vorteilhaft mit einem Streptavidin-Enzymconjugat, wie etwa Meerrettichperoxidase, nachgewiesen wird. Das Substrat der Enzymreaktion wird derart ausgewählt, daß sich an der Stelle der molekularen Wechselwirkung ein intensives, dunkles Prezipitat bildet, das den Detektionslaser quentscht. Daneben kann der Nachweis auch durch direkte Derivatisierung eines Wechselwirkungspartners mit einem Fluorochrom in Kombination mit einem geeigneten Laser erfolgen.

### Beispiel 1.1

Der Rezeptor (A) ist ein RNA-Aptamer, ein Peptid oder ein natürliches oder synthetisches Effektormolekül und der Analyt (B) ist ein Protein.

Die Eigenschaften eines Enzyms oder Regulationsfaktors bei der Genexpression werden häufig durch die Assoziation kleiner Effektor- oder Ligandmoleküle moduliert. Ein Beispiel sind Hormonrezeptoren, die nach Bindung an das Hormon in eine aktive Konformation überführt werden. Die Proteinfunktion kann durch Wechselwirkung eines Substratanalogons oder durch allosterische Effektoren moduliert werden. Um geeignete kleine Chemikalien zu identifizieren, werden Screening-Verfahren für Liganden durchgeführt, wobei eine große Anzahl an verschiedenen Chemikalien untersucht wird.

Mit dem erfindungsgemäßen Verfahren ist es möglich, chemische Bibliotheken durchzutesten. Dabei wird eine Vielzahl von verschiedenen kleinen Chemikalien in einer definierten Ordnung auf dem Meßträger immobilisiert, wobei das fragliche Protein unter verschiedenen Stringentbedingungen damit in Kontakt gebracht wird. Gebundenes Protein wird, wenn es mit einem geeigneten Fluorochrom, beispielsweise einem Latexpartikel, markiert ist, direkt nachgewiesen oder indirekt unter Verwendung einer Enzymamplifikationskaskade. In entsprechender Weise können große Bibliotheken von RNA-Aptameren oder Peptiden gescreent werden, entweder isoliert oder in ein Fusionsprotein eingebunden, hinsichtlich Wechselwirkungen mit einem in Frage stehenden Protein.

### Beispiel 1.2

Der Rezeptor (A) ist ein Protein und der Analyt (B) ist eine Nukleinsäure oder ein Ligant.

Proteinbibliotheken können hinsichtlich bestimmter Bindeeigenschaften, z. B. der Wechselwirkung mit einer definierten DNA-Sequenz gescreent werden. Vervielfältigte Meßträger, die definierte Bereiche von Proteinen enthalten, die zusammen die Produkte einer cDNA-Expressionsbibliothek darstellen, können mit spezifischen Liganten oder DNA-Bindestellen charakterisiert werden. Die angeordneten Proteine können so, wie sie in der Bibliothek sind, unbekannt sein und erst durch das erfindungsgemäße Verfahren zugeordnet werden. Alternativ können Bereiche von bekannten Proteinen oder Derivate eines definierten Proteins, wie etwa das Produkt einer zufälligen Mutagenese, untersucht werden.

### Beispiel 1.3

Der Rezeptor (A) ist eine DNA oder RNA und der Analyt (B) ist ein Protein.

Eine große Anzahl von verschiedenen doppelsträngigen DNA-Fragmenten kann auf den Nachweisfeldern aufgebracht werden. Dabei kann es sich entweder um kleine Fragmente, beispielsweise um Oligonukleotide oder um größere DNA-Fragmente bis zu sehr großen DNA-Abschnitten handeln, die eine geordnete Bibliothek von genomischen DNA-Fragmenten darstellen. Die Anwendungen umfassen die systematische Suche nach möglichen genomischen Bindestellen für DNA-Bindeproteine, aber auch andere DNA-Bindemoleküle können nachgewiesen werden, wie etwa Interkalatoren, kleine Moleküle mit Bevorzugung bestimmter Sequenzen, PNAs und Sequenzen, die eine Trippelhelix bilden. Ebenso können als Rezeptormoleküle in den Nachweisfeldern RNA-Moleküle aufgebracht werden, wie etwa die Transskripte einer cDNA-Bibliothek oder die Derivate einer definierten RNA, wie etwa das Produkt einer zufälligen Mutagenese.

### Beispiel 2

### Nukleinsäureuntersuchungen

Der Rezeptor (A) ist eine einzelsträngige DNA und der Analyt (B) ist eine einzelsträngige DNA oder RNA.

### Beispiel 2.1

### DNA-Kartierung

Es ist inzwischen möglich, ganze Genome zu sequenzieren und die Genome von höheren Eukarionten, wie etwa Menschen, Mäusen und Fliegen, werden kartiert und durch geordnete Klone (wie etwa P1-Phagenklone) abgedeckt, wobei davon ausgegangen wird, daß die Sequenzen aller Genome in absehbarer Zeit erhältlich sind. Hier können erfindungsgemäße Meßträger hergestellt werden, die ganze Genome in geordneten Arrays oder Bereichen enthalten. Damit ist es möglich, ein kloniertes Stück DNA in einem einzigen Hybridisierungsschritt seiner genomischen Lokalisierung zuzuordnen und abhängig von der Auflösung des Arrays (weiche eine Funktion der mittleren Sequenzlänge und der Zahl an einzelnen DNA-Molekülen ist) sogar das Gen selbst zu identifizieren. Ein Beispiel einer solchen Anwendung sind die P1-Arrays von Genome Systems, welche bisher schlecht zu screenen sind, da sie nur auf Membranen erhältlich sind. Ein Aufbringen dieser P1-Arrays auf den erfindungsgemäßen Meßträgern ermöglicht eine einfache und unkomplizierte Anwendung. Translokationen und andere genomische Umordnungen, die häufige Ursache genetischer Erkrankungen sind, können ebenfalls auf einfache Weise kartiert werden, einschließlich Deletionen des mitochondrialen Genoms. Darüber hinaus können Insertionen von Transgenen leicht kartiert werden, wenn die insertierte DNA zusammen mit einer kleinen Menge an flankierender genomischer DNA nachgewiesen wird.

### Beispiel 2.2

### Genidentifizierung und/oder Genklonen

Arrays oder Bereiche von diagnostischen Oligonukleotiden, die alle bekannten Gene einer gegebenen Spezies darstellen, ermöglichen die direkte Identifizierung eines in Frage stehenden Gens. Hierzu können z. B. cDNA-Arrays von Klontech oder die DNA-Chips von Affimetrix, auf denen alle Hefegene aufgetragen sind, durch Übertragung auf die erfindungsgemäßen Meßträger verbessert werden.

### Beispiel 2.3

### Expressions-Profiluntersuchungen

Durch Aufbringen von DNA auf erfindungsgemäße Meßträger, die alle Gene eines Organismus zeigen, kann das Expressionsprofil untersucht werden. Auf den Meßträger wird ein komplexes Gemisch von RNA aufgebracht, die den Expressionszustand einer bestimmten Zelle darstellen oder eine davon abgeleitete cDNA-Population. Der auf dem Meßträger festgestellte Expressionszustand kann leicht mit dem Expressionszustand anderer Zellen verglichen werden, die von anderen Gewebe stammen, andere Entwicklungsstadien oder andere Stoffwechselstadien darstellen. Alternativ können DNA-Proben untersuchtwerden, die bestimmte ausgewählte DNA-Zustände darstellen, wie etwa Zellzyklusgene, Signalübermittlerkomponenten usw. Da die erfindungsgemäßen Meßträger über lange Zeit gelagert werden können, können Expressionsprofile erstellt werden, die archiviert und zu Vergleichszwecken herangezogen werden können.

### Beispiel 2.4

### Molekulare Diagnose von DNA-Polymorphismen

Das erfindungsgemäße Verfahren kann zur Diagnose von üblichen und seltenen DNA-Polymorphismen verwendet werden, einschließlich der Kartierung von Mutationen in Proto-Onkogenen, die übliche Tumore hervorrufen. Ein geeigneter Meßträger enthält überlagernde Oligonukleotide, die zusammen ein gesamtes Gen (z. B. die Wildtypsequenz) umfassen, zusammen mit Oligonukleotiden, die alle möglichen oder häufig auftretende Mutationen dieser Sequenz enthalten. Das entsprechende DNA-Fragment wird von Patienten durch PCR isoliert und an den relevanten Gen-Meßträger unter Bedingungen hybridisiert, unter denen die Hybridisierung nur bei einer perfekten Sequenzübereinstimmung stattfindet. Ein Vergleich der Hybridisierung der experimentellen DNA an Wildtyp- bzw. Mutantoligonukleotide ermöglicht es, exakt festzustellen, welche Art von Mutation in einer Sequenz auftritt.

### Beispiel 3

Neben den oben beschriebenen Anwendungen, bei denen ein einfacher Bindevorgang nachgewiesen wird, der Signale ergibt, die ggf. durch eine Enzymkaskade verstärkt werden können, um die Sensitivität der Detektion zu erhöhen, kann das erfindungsgemäße Verfahren auch in Anwendungen verwendet werden, die umfangreichere Behandlungen des Meßträgers erfordern, wie etwa eine PCR-Amplifizierung, die analog zu in situ PCR-Amplifikationen durchgeführt wird. Auf diese Weise können auch infizierende Mittel nachgewiesen werden.

## Patentansprüche

1. Verfahren zum Nachweis von Analyten in einer Meßprobe, bei dem auf einem scheibenförmigen Substrat (3) auf einer seiner Flachseiten analytspezifische Binder (15) in einer Vielzahl von Nachweisfeldern (5, 7) immobilisiert werden, sodann die Meßprobe in Kontakt mit den Nachweisfeldern (5, 7) gebracht wird und anschließend das Vorhandensein oder/und die Menge der nachzuweisenden Analyten (17) durch optische Auswertung der Nachweisfelder (5, 7) ermittelt wird, wobei ein aus einem optisch transparenten Material gefertigtes Substrat (3) verwendet wird und die Nachweisfelder (5, 7) längs mindestens einer Spirallinie (27) oder/und längs einer Vielzahl konzentrischer Kreislinien verteilt auf dem Substrat (3) angeordnet werden, **dadurch gekennzeichnet, daß** nach Inkontaktbringung der Meßprobe mit den Nachweisfeldern (5, 7) eine optische Reflexionsschicht (21) auf der die Nachweisfelder (5, 7) tragenden Flachseite des Substrats (3) über den Nachweisfeldern (5, 7) aufgebracht wird.

2. Verfahren nach Anspruch 1,
**dadurch gekennzeichnet, daß** die Reflexionsschicht (21) aus Aluminium gebildet wird.

3. Verfahren nach einem der Ansprüche 1 und 2,
**dadurch gekennzeichnet, daß** bezogen auf die Scheibenachse radial benachbarte Nachweisfelder (5, 7) mit radialem Abstand voneinander angeordnet werden.

4. Verfahren nach einem der Ansprüche 1 bis 3,
**dadurch gekennzeichnet, daß** entlang der Spirallinie (27) bzw. einer Kreislinie einander benachbarte Nachweisfelder (5, 7) mit Abstand voneinander angeordnet werden.

5. Verfahren nach einem der Ansprüche 1 bis 4,
**dadurch gekennzeichnet, daß** auf der die Nachweisfelder (5, 7) tragenden Flachseite des Substrats (3) entlang der Spirallinie (27) bzw. mindestens einer Kreislinie zusätzlich Datenfelder (9) ausgebildet werden, welche meßprobenbezogene oder/und nachweisfeldbezogene oder/und auswertungsbezogene Informationen enthalten.

6. Verfahren nach Anspruch 5,
**dadurch gekennzeichnet, daß** Nachweisfelder (5, 7) und Datenfelder (9) abwechselnd entlang der Spirallinie (27) bzw. mindestens einer Kreislinie angeordnet werden.

7. Verfahren nach Anspruch 5,
**dadurch gekennzeichnet, daß** Nachweisfelder und Datenfelder jeweils auf gesonderten Kreislinien ausgebildet werden.

8. Verfahren nach einem der Ansprüche 5 bis 7,
**dadurch gekennzeichnet, daß** zur Bildung der Datenfelder (9) Vertiefungen (11) in die die Nachweisfelder (5, 7) tragende Flachseite des Substrats (3) eingebracht werden und daß die Reflexionsschicht (21) so aufgebracht wird, daß sie in die Vertiefungen (11) hineinreicht.

9. Verfahren nach einem der Ansprüche 5 bis 7,
**dadurch gekennzeichnet, daß** zur Bildung der Datenfelder eine einfallendes Leselicht beeinflussende Substanz auf die die Nachweisfelder tragende Flachseite des Substrats aufgebracht wird.

10. Verfahren nach einem der Ansprüche 1 bis 9,
**dadurch gekennzeichnet, daß** auf der die Nachweisfelder (5, 7) tragenden Flachseite des Substrats (3) entlang der Spirallinie (27) bzw. mindestens einer Kreislinie zusätzlich mindestens ein Referenzfeld ausgebildet wird, dessen optische Eigenschaften als Referenz bei der Auswertung der Nachweisfelder (5, 7) verwendet werden.

11. Verfahren nach einem der Ansprüche 1 bis 10,
**dadurch gekennzeichnet, daß** nach Inkontaktbringung der Meßprobe mit den Nachweisfeldern (5, 7) vor Aufbringung der Reflexionsschicht (21) eine Deckschicht (19) aus einem optisch transparenten Material auf die Nachweisfelder (5, 7) aufgebracht wird.

12. Verfahren nach Anspruch 11,
**dadurch gekennzeichnet, daß** für die Deckschicht (19) ein Material auf Polymerbasis verwendet wird.

13. Verfahren nach einem der Ansprüche 1 bis 12,
**dadurch gekennzeichnet, daß** ein Substrat (3) aus Polycarbonat verwendet wird.

14. Verfahren nach einem der Ansprüche 1 bis 13,
**dadurch gekennzeichnet, daß** das Substrat (3) an einem Herstellungsort mit den Bindern (15) versehen, getrocknet und verpackt wird und daß das so präparierte Substrat (3) sodann zu einem von dem Herstellungsort entfernten Anwendungsort gebracht wird, an dem die Meßprobe von einem Anwender in Kontakt mit den Nachweisfeldern (5, 7) gebracht wird.

15. Verfahren nach einem der vorhergehenden Ansprüche,
**dadurch gekennzeichnet, daß** der Nachweis der Analyten durch eine Detektion einer Änderung der optischen Eigenschaften der Nachweisfelder erfolgt.

16. Verfahren nach Anspruch 15,
**dadurch gekennzeichnet, daß** die optische Änderung der Nachweisfelder durch Isotope, Enzyme, Fluorochrome, Farbstoffe, Metallkolloide oder/und Beads herbeigeführt wird.

17. Verfahren nach Anspruch 16,
**dadurch gekennzeichnet, daß** Latex-Beads, Kunststoff-Beads, Glas-Beads oder/und Metall-Beads verwendet werden.

18. Meßträger zur Verwendung bei dem Verfahren nach einem der Ansprüche 1 bis 17,
umfassend ein scheibenförmiges, aus einem optisch transparenten Material gefertigtes Substrat (3), auf dem auf einer seiner Flachseiten analytspezifische Binder (15) in einer Vielzahl von Nachweisfeldern (5, 7) immobilisiert sind, wobei die Nachweisfelder (5, 7) längs mindestens einer Spirallinie (27) oder/und einer Vielzahl konzentrischer Kreislinien verteilt auf dem Substrat (3) angeordnet sind, **gekennzeichnet durch** eine nach Inkontaktbringung der Meßprobe mit den Nachweisfeldern auf der die Nachweisfelder tragenden Flachseite der Substrats flächig über den Nachweisfeldern (5, 7) aufzubringende Reflexionsschicht (21).

19. Meßträger nach Anspruch 18,
**dadurch gekennzeichnet, daß** auf der Reflexionsschicht (21) eine Schutzschicht (25) flächig aufgebracht ist.

20. Meßträger nach Anspruch 19,
**dadurch gekennzeichnet, daß** die Schutzschicht (25) aus einem Material auf Acrylbasis gefertigt ist.

21. Meßträger nach einem der Ansprüche 18 bis 20,
**dadurch gekennzeichnet, daß** er vor Aufbringung der Reflexionsschicht als verpackte Handelseinheit bereitgestellt ist.

22. Meßträger nach Anspruch 21,
**dadurch gekennzeichnet, daß** er getrocknet verpackt ist.

23. Verfahren zum Nachweis von Analyten in einer Meßprobe, bei dem auf einem scheibenförmigen Substrat auf mindestens einer seiner Flachseiten analytspezifische Binder in einer Vielzahl von Nachweisfeldern immobilisiert werden, sodann die Meßprobe in Kontakt mit den Nachweisfeldern gebracht wird und anschließend das Vorhandensein oder/und die Menge der nachzuweisenden Analysen durch Auswertung der Nachweisfelder ermittelt wird,
wobei die Nachweisfelder magnetisch ausgewertet werden und hierzu Binder oder die nachzuweisenden Analyten mit magnetischen oder/und magnetisierbaren Markern markiert werden und die Nachweisfelder längs einer Vielzahl konzentrischer Kreislinien oder/und längs mindestens einer Spirallinie verteilt auf dem Substrat angeordnet werden,
**dadurch gekennzeichnet, daß** vor der Ausbildung der Nachweisfelder oder nach Inkontaktbringung der Meßprobe mit den Nachweisfeldern eine magnetische oder/und magnetisierbare Partikel enthaltende Magnetschicht flächig über jeder Nachweisfelder tragenden Flachseite des Substrats aufgebracht wird.

24. Verfahren nach Anspruch 23,
**dadurch gekennzeichnet, daß** nach Inkontaktbringung der Meßprobe mit den Nachweisfeldern eine Fixierschicht auf die Nachweisfelder aufgebracht wird.

25. Verfahren nach Anspruch 24,
**dadurch gekennzeichnet, daß** die Fixierschicht flächig auf jede Nachweisfelder tragende Flachseite des Substrats aufgebracht wird.

26. Verfahren nach Anspruch 24 oder 25,
**dadurch gekennzeichnet, daß** für die Fixierschicht ein Material auf Polymerbasis verwendet wird.

27. Verwendung eines Meßträgers nach einem der Ansprüche 18 bis 22 in einem Immunoassay oder/und Nukleinsäurehybridisierungsassay oder/und Lektin-Zucker-Assay oder/und Protein-Nukleinsäureassay.

## Claims

1. Method for the detection of analytes in a sample, wherein analyte-specific binders (15) are immobilised in a large number of detection fields (5, 7) located on one of the planar faces of a disc-shaped substrate (3), then the sample is brought into contact with the detection fields (5, 7), and subsequently the presence and/or the quantity of the analytes (17) to be detected is (are) determined by optical evaluation of the detection fields (5,7), wherein a substrate (3) prepared from an optically transparent material is used, and the detection fields (5, 7) are arranged along at least one spiral line (27) and/or a large number of concentric circular lines on the substrate (3), **characterised in that**, after contacting the sample with the detection fields (5, 7), an optical reflecting layer (21) - the planar face of the substrate (3) which carries the detection fields (5, 7) - is applied over the detection fields (5, 7).

2. Method according to claim 1, **characterised in that** the reflecting layer (21) is made of aluminium.

3. Method according to either of claims 1 and 2, **characterised in that**, with reference to the disc axis, radially adjacent detection fields (5, 7) are arranged with radial spacing from each other.

4. Method according to any of claims 1 to 3, **characterised in that**, along the spiral line (27) or a circular line, adjacent detection fields (5, 7) are arranged with spacing from each other.

5. Method according to any of claims 1 to 4, **characterised in that**, on the planar face of the substrate (3) which carries the detection fields (5, 7), along the spiral line (27), or along at least one circular line, additional data fields (9) are formed which contain data pertaining to samples and/or detection fields and/or the evaluation.

6. Method according to claim 5, **characterised in that** detection fields (5, 7) and data fields (9) are arranged alternately along the spiral line (27) or along at least one circular line.

7. Method according to claim 5, **characterised in that** detection fields and data fields are each formed on separate circular lines.

8. Method according to any of claims 5 to 7, **characterised in that** for the formation of the data fields (9), recesses (11) are formed in the planar face of the substrate (3) which carries the detection fields (5, 7), and **in that** the reflecting layer (21) is applied in such a manner that it reaches into the recesses (11) .

9. Method according to any of claims 5 to 7, **characterised in that** for the formation of the data fields, a substance which influences incident reading light is applied on the planar face of the substrate which carries the detection fields.

10. Method according to any of claims 1 to 9, **characterised in that**, on the planar face of the substrate (3) which carries the detection fields (5, 7), along the spiral line (27) or along at least one circular line, at least one reference field is formed, in addition, the optical properties of which are used as reference in the evaluation of the detection fields (5, 7).

11. Method according to any of claims 1 to 10, **characterised in that**, after contacting the sample with the detection fields (5, 7), before the application of the reflecting layer (21), a coating layer (19) made of an optically transparent material is applied to the detection fields (5, 7).

12. Method according to claim 11, **characterised in that** for the coating layer (19) a polymer-based material is used.

13. Method according to any of claims 1 to 12, **characterised in that** a substrate (3) made of polycarbonate is used.

14. Method according to any of claims 1 to 13, **characterised in that** the substrate (3) is provided at a manufacturing site with the binders (15), dried and packaged, and **in that** the substrate (3) so prepared is then brought to an application site which is at a distance from the manufacturing site, at which application site the sample is brought into contact by a user with the detection fields (5, 7).

15. Method according to any of the preceding claims, **characterised in that** the detection of the analytes is carried out by a detection of a change in the optical properties of the detection fields.

16. Method according to claim 15, **characterised in that** the optical change in the detection fields is caused by isotopes, enzymes, fluorochromes, dyes, metal colloids and/or beads.

17. Method according to claim 16, **characterised in that** latex beads, plastic beads, glass beads and/or metal beads are used.

18. Support for use with the method according to any of claims 1 to 17, comprising a disc-shaped substrate (3) made of an optically transparent material, on one of the planar sides of which analyte-specific binders (15) are immobilised in a large number of detection fields (5, 7), wherein the detection fields (5, 7) are arranged along at least one spiral line (27) and/or a large number of concentric circular lines on the substrate (3), **characterised by** a reflecting layer (21) being flatly applied, over the detection fields (5, 7), on the planar face of the substrate which carries the detection fields, after bringing the sample into contact with the detection fields.

19. Support according to claim 18, **characterised in that** a protective layer (25) is flatly applied on the reflecting layer (21).

20. Support according to claim 19, **characterised in that** the protective layer (25) is made of an acrylate-based material.

21. Support according to any of claims 18 to 20, **characterised in that** it is made available as a packaged commercial unit, prior to the application of the reflecting layer.

22. Support according to claim 21, **characterised in that** it is packaged in the dry state.

23. Method for the detection of analytes in a sample wherein analyte-specific binders are immobilised in a large number of detection fields on at least one of the planar faces of a disc-shaped substrate, then the sample is brought into contact with the detection fields, and subsequently the presence and/or the quantity of the analytes to be determined is (are) determined by evaluation of the detection fields, wherein the detection fields are magnetically evaluated and, for that purpose, binders, or the analytes to be detected, are labelled with magnetic and/or magnetisable labels and the detection fields are arranged along a large number of concentric circular lines and/or along at least one spiral line on the substrate, **characterised in that**, prior to the formation of the detection fields and after bringing the sample into contact with the detection fields, a magnetic layer, which contains magnetic and/or magnetisable particles, is flatly applied over each planar face of the substrate which carries detection fields.

24. Method according to claim 23, **characterised in that**, after bringing the sample into contact with the detection fields, a fixing layer is applied to the detection fields.

25. Method according to claim 24, **characterised in that** the fixing layer is flatly applied on each planar face of the substrate which carries detection fields.

26. Method according to claim 24 or 25, **characterised in that**, for the fixing layer, a polymer-based material is used.

27. Use of a support according to any of claims 18 to 22 in an immunoassay and/or nucleic acid hybridisation assay and/or lectin-sugar assay and/or protein-nucleic acid assay.

## Revendications

1. Procédé permettant la détection d'analytes dans un échantillon d'essai, pour lequel des liants (15) spécifiques aux analytes sont immobilisés sur un substrat en forme de disque (3) sur l'une de ses faces planes dans une multitude de champs de détection (5, 7) de sorte que l'échantillon d'essai soit mis en contact avec les champs de détection (5, 7) et qu'ensuite l'existence ou/et la quantité d'analytes à détecter (17) soit déterminée par une analyse optique des champs de détection (5, 7), un substrat (3) fabriqué à partir d'un matériau transparent d'un point de vue optique et dans lequel les champs de détection (5, 7) répartis sur le substrat (3) sont agencés le long d'au moins une ligne hélicoïdale (27) ou/et le long de nombreuses lignes circulaires concentriques étant utilisé, **caractérisé en ce que** l'on applique, après la mise en contact de l'échantillon d'essai avec les champs de détection (5, 7), une couche de réflexion optique (21) sur la face plane du substrat (3) supportant les champs de détection (5, 7), au dessus des champs de détection (5, 7).

2. Procédé selon la revendication 1, **caractérisé en ce que** la couche de réflexion (21) est formée à partir d'aluminium.

3. Procédé selon l'une quelconque des revendications 1 et 2, **caractérisé en ce que** les champs de détection (5, 7) proches radialement, en considérant le rayon par rapport à l'axe du disque, sont agencés avec un écart radial les uns par rapport aux autres.

4. Procédé selon l'une quelconque des revendications 1 à 3, **caractérisé en ce que** le long de la ligne hélicoïdale (27) resp. d'une ligne circulaire, les champs de détection (5, 7) proches les uns des autres sont agencés avec un écart les uns par rapport aux autres.

5. Procédé selon l'une quelconque des revendications 1 à 4, **caractérisé en ce** des champs de données supplémentaires (9) sont formés sur la face plane du substrat (3) supportant les champs de détection (5, 7) le long de la ligne hélicoïdale (27) resp. au moins d'une ligne circulaire, lesquels comprennent des informations relatives à l'analyse et/ou aux champs de détection et/ou à l'échantillon d'essai.

6. Procédé selon la revendication 5, **caractérisé en ce que** les champs de détection (5, 7) et les champs de données sont agencées de manière alternative le long de la ligne hélicoïdale (27) resp, au moins d'une ligne circulaire.

7. Procédé selon la revendication 5, **caractérisé en ce que** les champs de détection (5, 7) et les champs de données sont à chaque fois formés sur des lignes circulaires propres.

8. Procédé selon l'une quelconque des revendications 5 à 7 **caractérisé en ce que** l'on réalise des alvéoles (11) sur la face plane du substrat (3) supportant les champs de détection (5, 7) en vue de former les champs de données (9) et **en ce que** l'on applique la couche de réflexion (21) de telle sorte qu'elle pénètre dans les alvéoles (11).

9. Procédé selon l'une quelconque des revendications 5 à 7 **caractérisé en ce que** l'on applique sur face plane du substrat supportant les champs de détection, une substance influençant la lumière de lecture descendante en vue de former les champs de données.

10. Procédé selon l'une quelconque des revendications 1 à 9 **caractérisé en ce qu'**au moins un champ de référence supplémentaire est formé sur la face plane du substrat (3) supportant les champs de détection (5, 7) le long de la ligne hélicoïdale (27) resp. d'au moins une ligne circulaire, dont on utilise les propriétés optiques comme référence lors de l'analyse des champs de détection (5, 7).

11. Procédé selon l'une quelconque des revendications 1 à 10 **caractérisé en ce que** l'on applique, après la mise en contact du procédé de mesure avec les champs de détection (5, 7), et avant l'application d'une couche de réflexion optique, un revêtement (19) réalisé dans un matériau transparent d'un point de vue optique sur les champs de détection (5, 7).

12. Procédé selon la revendication 11, **caractérisé en ce que** l'on utilise un matériau à base de polymère pour le revêtement (19).

13. Procédé selon l'une quelconque des revendications 1 à 12 **caractérisé en ce que** l'on utilise un substrat (3) en polycarbonate.

14. Procédé selon l'une quelconque des revendications 1 à 13, **caractérisé en ce que** l'on dote le substrat avec des liants (15) sur le site de fabrication, qu'on le sèche et qu'on le conditionne et **en ce que** l'on apporte ensuite le substrat (3) ainsi préparé vers un site d'utilisation éloigné du site de fabrication, site sur lequel un utilisateur mettra en contact l'échantillon d'essai avec champs de détection (5, 7).

15. Procédé selon l'une quelconque des revendications précédentes **caractérisé en ce que** la détection des analytes est réalisée par une détection d'une modification des propriétés optiques des champs de détection.

16. Procédé selon la revendication 15, **caractérisé en ce que** la modification optique du champ de détection est réalisée par isotopes, enzymes, fluorochromes, colorants, colloïdes métalliques ou/et billes.

17. Procédé selon la revendication 16, **caractérisé en ce que** l'on utilise des billes de latex, des billes en matière plastique, des billes de verre ou/et des billes de métal.

18. Support de mesure pour l'utilisation dans le procédé selon l'une quelconque des revendications 1 à 17, comprenant un substrat en forme de disque (3) réalisé dans un matériau transparent d'un point de vue optique, sur lequel des liants (15) spécifiques aux analytes sont immobilisés sur l'une de ses faces planes dans une multitude de champs de détection (5, 7), les champs de détection (5, 7) répartis sur le substrat (3) étant agencés le long d'au moins une ligne hélicoïdale (27) ou/ et le long de nombreuses lignes circulaires concentriques, **caractérisé en ce que** l'on applique, après la mise en contact de l'échantillon d'essai avec les champs de détection, une couche de réflexion optique (21) sur la face plane du substrat (3) supportant les champs de détection (5, 7) de manière plane sur les champs de détection (5, 7).

19. , Support de mesure selon la revendication 18 **caractérisé en ce que** l'on applique de manière plane une couche protectrice (25) sur la couche de réflexion (21).

20. Support de mesure selon la revendication 19 **caractérisé en ce que** la couche protectrice (25) est réalisée dans un matériau à base d'acrylique.

21. Support de mesure selon l'une quelconque des revendications 18 à 20, **caractérisé en ce qu'**avant l'application de la couche de réflexion il est préparé sous forme d'unité commerciale conditionnée.

22. Support de mesure selon la revendication 21 **caractérisé en ce qu'**il est conditionné séché.

23. Procédé permettant la détection d'analytes dans un échantillon d'essai, pour lequel des liants (15) spécifiques aux analytes sont immobilisés sur un substrat en forme de disque sur au moins l'une de ses faces planes dans une multitude de champs de détection de sorte que l'échantillon d'essai soit mis en contact avec les champs de détection (5, 7) et qu'ensuite l'existence ou/ et la quantité d'analytes à détecter (17) soit déterminée par une analyse des champs de détection (5, 7), les champs de détection étant analysés de façon magnétique et par conséquent les liants ou les analytes à détecter étant marqués avec un marqueur magnétique ou magnétisable et les champs de détection répartis sur le substrat étant agencés le long de nombreuses lignes circulaires concentriques ou/et le long d'au moins une ligne hélicoïdale, **caractérisé en ce que** l'on applique de manière plane, avant la formation de champs de détection ou après la mise en contact de l'échantillon d'essai avec les champs de détection, une couche magnétique contenant des particules magnétiques ou/et magnétisables sur chacune des faces planes du substrat supportant les champs de détection.

24. Procédé selon la revendication 23, **caractérisé en ce que** l'on applique après la mise en contact de l'échantillon d'essai avec les champs de détection un couche de fixation sur les champs de détection.

25. Procédé selon la revendication 24, **caractérisé en ce que** l'on applique la couche de fixation de manière plane sur chacune des faces planes du substrat supportant les champs de détection.

26. Procédé selon la revendication 24 ou 25, **caractérisé en ce que** l'on utilise pour la couche de fixation un matériau à base de polymère.

27. Utilisation d'un support de mesure selon l'une quelconque des revendications 18 à 22 dans un immuno-essai ou/et dans un essai d'hybridation de l'acide nucléique, ou/et dans un essai lectine - sucre ou/et dans un essai acide nucléique - protéine.
